# EUROPEAN PATENT SPECIFICATION

(11) **EP 2 257 295 B1**
(45) Date of publication and mention of the grant of the patent: **25.03.2020**
(21) Application number: 08837807.0
(22) Date of filing: 07.10.2008
(51) Int. Cl.: A61K 31/5415, A61K 41/00, A61P 31/04, A61K 35/00

(54) **PHOTODYNAMIC THERAPY PROCESS AND PHOTOSENSITIZER COMPOSITIONS**
PHOTODYNAMISCHES THERAPIEVERFAHREN UND LICHTSENSIBILISATOR-ZUSAMMENSETZUNGEN
PROCÉDÉ DE THÉRAPIE PHOTODYNAMIQUE ET COMPOSITIONS PHOTOSENSIBILISANTES

(30) Priority: 08.10.2007 US 978219 P; 06.10.2008 US 245927
(43) Date of publication of application: 08.12.2010
(73) Proprietor: Ondine International Ltd., St. Michael (BB)
(72) Inventor: LOEBEL, Nicholas, Woodinville WA 98072 (US); STREET, Cale, Edmonds WA 98026 (US)
(74) Representative: Geary, Stephen
(86) International application number: PCT/US2008/079050
(87) International publication number: WO 2009/048868

(56) References cited:
- WO-A-95/32732
- WO-A-98/28607
- WO-A-02/096896
- WO-A-2006/034219
- WO-A-2006/127482
- WO-A-2006/135344
- JP-A- 49 020 323
- WAINWRIGHT M ET AL: "A STUDY OF PHOTOBACTERICIDAL ACTIVITY IN THE PHENOTHIAZINIUM SERIES" FEMS IMMUNOLOGY AND MEDICAL MICROBIOLOGY, ELSEVIER SCIENCE B.V., AMSTERDAM, NL, vol. 19, no. 1, 1 September 1997 (1997-09-01), pages 75-80, XP001042388 ISSN: 0928-8244
- WAINWRIGHT M ET AL: "PHOTOBACTERICIDAL ACTIVITY OF PHENOTHIAZINIUM DYES AGAINST METHICILLIN-RESISTANT STRAINS OF STAPHYLOCOCCUS AUREUS" FEMS MICROBIOLOGY LETTERS, AMSTERDAM, NL, vol. 160, no. 2, 15 March 1998 (1998-03-15), pages 177-181, XP001042387 ISSN: 0378-1097
- MEINDL W ET AL: "[Antimycobacterial properties of phenothiazine type compounds]" ARCHIV DER PHARMAZIE MAR 1989, vol. 322, no. 3, March 1989 (1989-03), pages 133-136, XP002508934 ISSN: 0365-6233
- DATABASE MEDLINE [Online] US NATIONAL LIBRARY OF MEDICINE (NLM), BETHESDA, MD, US; September 1994 (1994-09), EFFROS R M ET AL: "Reduction and uptake of methylene blue from rat air spaces." XP002508945 Database accession no. NLM7836153 & JOURNAL OF APPLIED PHYSIOLOGY (BETHESDA, MD. : 1985) SEP 1994, vol. 77, no. 3, September 1994 (1994-09), pages 1460-1465, ISSN: 8750-7587
- MERKER M P ET AL: "Pulmonary endothelial thiazine uptake: separation of cell surface reduction from intracellular reoxidation." THE AMERICAN JOURNAL OF PHYSIOLOGY APR 1997, vol. 272, no. 4 Pt 1, April 1997 (1997-04), pages L673-L680, XP008099903 ISSN: 0002-9513
- DATABASE MEDLINE [Online] US NATIONAL LIBRARY OF MEDICINE (NLM), BETHESDA, MD, US; July 1995 (1995-07), BONGARD R D ET AL: "Reduction of thiazine dyes by bovine pulmonary arterial endothelial cells in culture." XP002508946 Database accession no. NLM7631818 & THE AMERICAN JOURNAL OF PHYSIOLOGY JUL 1995, vol. 269, no. 1 Pt 1, July 1995 (1995-07), pages L78-L84, ISSN: 0002-9513
- MAY JAMES M ET AL: "Reduction and uptake of methylene blue by human erythrocytes." AMERICAN JOURNAL OF PHYSIOLOGY. CELL PHYSIOLOGY JUN 2004, vol. 286, no. 6, June 2004 (2004-06), pages C1390-C1398, XP002508938 ISSN: 0363-6143

## Description

### TECHNICAL FIELD

The present invention relates to a compound for use in a photodynamic therapy process and a photosensitizer composition useful for that therapy. More particularly, the present invention relates to a photosensitizer composition having photoactive ingredients in a form that is more suitable for transport to a target medium and/or provides for more efficient light absorption.

### BACKGROUND OF THE INVENTION

Photodynamic therapy, particularly photodynamic disinfection, has been demonstrated to be an effective non-antibiotic antimicrobial approach *in vitro.* For the purpose of this specification, photodynamic therapy and photodynamic disinfection shall hereinafter be collectively referred to as photodynamic therapy. One exemplary advantage of photodynamic therapy as an antimicrobial treatment modality is that it is typically not subject to issues of resistance that can plague the use of antibiotics. As another exemplary advantage, it can be employed as a localized topical treatment that can be administered in areas such as the oral cavity where reliable topical antibiotic delivery can be problematic. For these reasons and others, photodynamic therapy is fast becoming a valuable tool in the treatment of infection process and/or bacterial-related conditions such as periodontal disease.

Photodynamic therapy fundamentally involves the use of light energy to activate one or more photoactive ingredients of a photosensitizer composition so that those ingredients can then either pass energy on directly to a substrate/target (type I reaction), or can interact with molecular oxygen to produce reactive oxygen species (type II reaction). These reactions mediate the non-specific inhibition of microbial, certain human (e.g. tumor) cells and other pathogens such as virus primarily via lipid peroxidation, membrane damage, and damage to intracellular components. In order for this process to generate an antimicrobial (e.g., bactericidal) or other desired effect, it is typically desirable that the photosensitizer composition, and particularly the photoactive ingredients thereof, be internalized into or brought into very close association with the cell wall/membrane, cytoplasm, and/or inner constituents of a target cell or organism.

The molecular properties of a photosensitizer composition and/or its ingredients can play a large role in determining the site of action and therefore the antimicrobial (e.g., bactericidal) mechanism. Specifically, the charge and lipophilicity ratio of the composition and its ingredients can play a significant role. Some photoactive ingredients, such as methylene blue, are polar in nature and hence are very soluble in an aqueous environment. Ingredients or molecules of this type can remain in solution either intra- or extracellularly, and may demonstrate relatively little physical association or integration with or into a target medium (e.g., may not fractionate or associate with hydrophobic targets) such as bacterial membranes or cells. Moreover, it can often be the case that these photoactive ingredients are passed through aqueous trans-membrane channels (e.g., passive diffusion into the cytoplasm) or be brought across in aqueous vesicles via an active transport process into the intracellular environment of the bacteria or other target. Thus, it can be difficult for these ingredients to become closely associated with the cell wall/membrane of a target organism.

On the other hand, non-polar photoactive ingredients with hydrophobic properties are able to integrate into the bacterial membrane via preferential association with the hydrophobic interior of the lipid bilayer. Photoactive ingredients of this type can thus associate with the outer bacterial membrane, or if internalized, can be partitioned into a hydrophobic location within the bacterial cell. Thus, non-polar photoactive ingredients can more easily associate with and pass through the cell wall/membrane of a target organism, such as a bacterium or other microbe.

Association with cell walls or membranes can be further complicated by the fact that Gram-negative and Gram-positive bacteria differ in structure. Gram-positive strains, such as *Staphylococcus aureus ("S. aureus")*, possess a relatively permeable cell wall consisting largely of peptidoglycan. By contrast, Gram-negative strains such as *E. Coli* have two overlapped walls, each consisting of a separate phospholipid bilayer with the outer wall or membrane containing a large proportion of lipopolysaccharide (LPS) on the outer surface (often accounts for ∼40% of the mass of the outer membrane), rendering such cells much less permeable. While both strains have a net negative charge on the outer surface, the significant number of negatively charged molecular groups comprising LPS also confers a large overall negative charge on the Gram-negative strains. Thus, photosensitizer molecules or ingredients that are cationic (i.e., positively charged) exhibit enhanced uptake at bacterial surfaces (due to electrostatic or opposite charge attraction) compared to anionic or neutral species, although uptake/internalization is generally greater in Gram-positive strains than Gram-negative strains simply due to the physical barrier structure involved.

A recent study (Maisch et al, 2007) used singlet oxygen detection techniques to show that PHOTOFRIN®, a commercial photosensitizer composition, associates with and has a photocatalytic site of action at or in the bacterial membrane of *S*. *aureus.* Another study (Tegos and Hamblin, 2006) examined the ability of bacterial multidrug resistance pumps to remove photosensitizers such as methylene blue, toluidine blue O, and dimethylmethylene blue from the intracellular environment, thus decreasing the efficacy of photodynamic therapy. Their results suggest that these photoactive ingredients are actively taken up into the strains examined (both Gram-negative and Gram-positive) and that the site of action is within the intracellular environment. Since the intracellular environment is unique and is closely regulated across various physiological parameters (osmolarity, pH, alkalinity, charge, redox, etc), it would be desirable to control photoactive molecules or ingredients such that they behave in a certain manner in an extracellular situation but exhibit different behavior once internalized with the intent of increasing the efficacy of the photodynamic reaction. The present invention provides such differing behaviors.

WO2006/135344 describes a photosensitizing composition comprising a mixture of a surfactant, an alcohol, and/or water with a ratio of these components of 10:5:85 to 40:30:30 and in a preferred embodiment includes a photosensitizing compound with a mixture of glycerol, ethanol and water. WO02/096896 discloses that certain methylene blue derivatives are photodynamic agents for treating cancer and bacterial infections. WO2006/034219 describes a method for inactivation of microorganisms through contact with phenothiazinium and an inhibitor and irradiating the phenothiazinium. WO98/28607 describes a pharmaceutical composition containing methylene blue and a reducing agent for inactivation of viruses by irradiation. Wainwright et al "A Study of Photobactericidal Activity in the Phenothiazinium Series" FEMS Immunology and Medical Microbiology Vol 19 No 1 1 September 1997 pages 75-80 describes the antibacterial activity of toluidine blue and methylene blue. Wainwright et al "Photobactericidal Activity of Phenothiazinium Dyes against Methicillin-ResitantStrains of Staphylococcus Aureus" FEMS Microbiology Letters Vol 160 No 2 15 March 1998 pages 177-181 describes tests of a series of phenothiazinium dyes against various strains of Staphylococcus aureus. Meindl et al "Antimycobacterial Properties of Phenothiazine Type Compounds" Archiv der Pharmazie Mar 1989 Vol 322 No 3 pages 133-136 discloses that phenothiazines ae anti mycobacterial agents. WO95/32732 describes adding methylene blue to a preparation containing ascorbate to inactivate viruses in the preparation by irradiation. WO2006/27482 discloses the use of methylene blue ascorbate in photodynamic therapy of hepatitis. JP49 020323 describes a composition containing methylene blue and ascorbic acid.

### SUMMARY OF THE INVENTION

The present invention provides use of a photosensitizer composition in the manufacture of a medicament for inhibiting at least one Gram-negative bacterial cell during photodynamic therapy comprising:
providing a photosensitizer composition having a phenothiazine in a reduced state wherein the phenothiazine in the reduced state has lost a degree of color and at least 10% of optical density compared to the phenothiazine in a photoactive state;
applying the photosensitizer composition to a substrate containing the at least one Gram-negative bacterial cell such that the phenothiazine in the reduced state associates with at least one of the following: bacterial membrane structure of the at least one Gram-negative bacterial cell, intracellular environment of the at least one Gram-negative bacterial cell, and a combination thereof, and becomes oxidized to the photoactive state; and
exposing the phenothiazine to light at a wavelength absorbed by the phenothiazine so that the phenothiazine at least assists in inhibiting the at least one Gram-negative bacterial cell.

In one embodiment the photosensitizer composition may be employed in a method for photodynamic disinfection comprising: providing the photosensitizer composition having a phenothiazine in a reduced state; applying the photosensitizer composition to a substrate such that the phenothiazine associates with a target medium; allowing the phenothiazine to reach a photoactive state while at the location adjacent, contacting or taken within (collectively hereinafter referred to as "adjacent") the target medium; exposing the phenothiazine to light at a wavelength absorbed by the phenothiazine so that the phenothiazine at least assists in inhibiting at least one pathogen associated with, or comprising, the target medium. The term "pathogen" as used herein shall mean undesired prokaryotic or eukaryotic cells or collections of cells, including microbes, bacteria, virus, fungi, tumor cells, or other nucleic-acid containing particles or cells or. The terms "inhibiting" "inhibition" and/or "inhibit" as used herein shall mean to inhibit, reduce, destroy, kill and/or eliminate.

### BRIEF DESCRIPTION OF THE DRAWINGS

FIG. 1 is a graph showing optical absorbance measurements for an oxidized methylene blue solution and also for several reduced methylene blue solutions using 0.05% w/v, 0.1% w/v, 0.2% w/v, 0.5% w/v, and 1.0% w/v ascorbic acid as the reducing agent as described in Example I;
FIG. 2 is a bar graph showing the antimicrobial efficacy data of a reduced methylene blue solution and a non-reduced methylene blue solution in the photodynamic disinfection of *E*. *coli* versus controls as described in Example II; and
FIG. 3 is a table showing the antimicrobial efficacy data of various samples used in the experiment as described in Example III.

### DESCRIPTION OF THE PREFERRED EMBODIMENT

The present invention is predicated upon provision of a process for performing photodynamic therapy upon tissue or other substrates and a photosensitizer composition suitable for use in the process. As used herein, the term "tissue" can include any substrate associated with a collection of cells (e.g., a collection of bacterial cells or a localized infection or tumor site). Typically, the process of the present invention includes provision of the photosensitizer compositions with photoactive ingredients in a chemically reduced state. The term "chemically reduced state" shall mean a partially reduced state and/or a wholly reduced state with respect to the neutral point of the molecule or composition as part of a redox couple. The reduced state of the photoactive ingredients can render the ingredient more transportable and/or suitable for passage through one or more barrier mediums such that the photosensitizer composition, particularly the photoactive ingredients, can more effectively reach a target medium. At the target medium, the photoactive ingredients are typically converted to a second state (e.g., a photoactive state) that is particularly suitable for photodynamic therapy. The reduced state can also provide the ingredients in a relatively colorless or low color state prior to conversion of the ingredients to the second state (i.e., a colored, oxidized state) such that photodynamic therapy can be more efficiently or selectively directed at a target medium without causing the same amount of undesirable non-specific staining, killing and/or unnecessary light absorption caused by other photosensitizer compositions. Moreover, this colorless or low color state can cause such efficiencies and selectivity regardless of whether the photoactive ingredients pass through a barrier medium or are placed directly in apposition to the target medium.

### Photosensitizer Composition

The photosensitizer composition can include at least one photoactive ingredient and optionally a combination of the at least one photoactive ingredient and other ingredients. Such other ingredients can include, without limitation, solvents (aqueous or otherwise), buffering components (osmolarity, pH), diluent, adjuvant, excipient, and redox-potential-controlling agents, viscosity agents, surfactants, flavorants, preservatives, cell permeabilizing agents, antibiotics, bactericides/bacteriostats, and combinations thereof. The ingredients are preferably approved by a regulatory agency of the Federal or a State government, or listed in the U.S. Pharmacopeia or other generally recognized pharmacopeia for use in animals, and more particularly in humans.

Generally, the at least one photoactive ingredient is capable of exhibiting photoactive characteristics. As used herein, the term photoactive ingredient encompasses ingredients that are photoactive or that can become photoactive as will be understood further below. Preferably, the photoactive ingredients can assist in the performance of photodynamic therapy, and particularly the inhibition of undesirable pathogens, cells or the like according to one or multiple different mechanisms. According to one mechanism, the one or more photoactive ingredients are activated upon exposure to light energy such that the one or more ingredients can pass that energy to and/or engage in redox reactions with a target medium and particularly cells (e.g., bacterial cells) of that target medium in order to inhibit those cells. According to another mechanism, the one or more photoactive ingredients are activated upon exposure to light energy such that the photoactive ingredients interact with molecular oxygen to produce reactive oxygen species that are toxic to the undesired cells.

The photoactive ingredient is from the phenothiazine class (e.g., methylene blue and its derivatives, or toluidine blue).

Depending on the desired application, the photosensitizer composition may optionally comprise a plurality of the photoactive ingredients. The amount or concentration of the photoactive ingredient(s) may vary depending upon the desired application, the particular photoactive ingredient(s) used, and the target microbes to be destroyed. For example, concentration of the photoactive ingredient(s) in the photosensitizer composition may range from about 0.00001% to about 25% w/v, from about 0.0001% to about 10% w/v, from about 0.001% to about 1% w/v, from about 0.001% w/v to about 0.1% w/v, or from about 0.005% w/v to about 0.05% w/v.

It is contemplated that the light or light energy of the present invention can be provided by any electromagnetic radiation source including visible light and non-visible radiation or light. The light can contain any wavelength(s) that can be absorbed by the at least one photoactive ingredient of the photosensitizer composition, when the at least one photoactive ingredient is in a colored and/or photoactive state. The wavelengths include wavelengths selected from the continuous electromagnetic spectrum, ranging from below the ultraviolet ("UV") range, through the visible range, and into and beyond the near, mid and far-infrared range etc. The wavelengths are generally preferably between about 160 nm to about 1600 nm, more preferably between about 400 nm to about 800 nm, most preferably between about 500 nm to about 850 nm although the wavelengths may vary depending upon the particular photoactive ingredient(s) used and the light intensity. The light may be produced by any suitable art-disclosed light emitting devices such as lasers, light emitting diodes ("LEDs" including organic light emitting diodes ["OLED's"], superluminescent light emitting diodes ["SLED's")], or the like), incandescent sources, fluorescent sources, or the like.

At least one and possibly all of the photoactive ingredients are capable of being altered to a state that make the ingredients more suitable for transport through one or more barrier mediums to one or more target mediums. In addition or alternatively, the altered state of the photoactive ingredients can make the ingredients less colored such that the ingredients can cause less staining, less non-specific (i.e., away from the desired treatment area) killing and/or less unnecessary light absorption (e.g., by allowing light energy to pass through the composition to the target site).

Although other alternative states may be employed for the photoactive ingredients, it is preferable for the photoactive ingredients to be reacted or otherwise brought to a chemically reduced state (i.e., a molecular state whereby the photoactive ingredient is chemically "reduced" via a gain of electrons from another substance which in turn donates said electrons, itself becomes oxidized, and thus acts as the "reducing agent"). Thus, it is preferable for the photoactive ingredient to be capable of being driven, either fully or partially, to a reduced state for transport through the barrier medium. Generally, it is contemplated that nearly any photoactive ingredient that can be reduced by virtue of a reduction reaction can be employed in the photosensitizer composition. Preferred photoactive ingredients are dyes and more preferably phenothiazine dyes or derivatives thereof such as methylene blue or toluidine blue O. It is to be understood that the term photoactive ingredients include the ingredients that are not necessarily "photoactive" in their fully reduced form but become "photoactive" after re-oxidation to a colored form.

In various biological systems, several key enzymes and major metabolic pathway reactions can mediate the oxidation or reduction of photoactive ingredients that are brought into association with a pathogen, unwanted cell or cluster of cells, organisms or pathogens. For example, there is evidence that a thiazine reductase enzyme present in the membrane of mammalian endothelial cells can reduce methylene blue to its colorless (*leuco*) form at the cell surface. In the reduced form, a photoactive ingredient such as a thiazine dye, being substantially uncharged and relatively lipophilic in nature after such reduction, can cross the cell membrane with little difficulty by a diffusion process or otherwise. Once in the cell, the photoactive ingredient is re-oxidized to the colored (photoactive) form by, for example, exposure to molecular oxygen, heme-containing proteins, active intracellular oxidation processes or otherwise. Thus, the reduced photoactive ingredient is more capable of crossing a biological barrier medium and more closely associating with the target medium (e.g., pathogen, cell, organism or combination thereof). Moreover, the colorless or substantially colorless nature of the reduced photoactive ingredient (as compared to the intense color observed in the oxidized form) will decrease incidental staining of tissue or otherwise and will allow activating light energy to more readily penetrate into the treatment site such that photodynamic reactions can be more efficiently driven. This, in turn, may allow for less light to be used for photodynamic therapy and/or inhibition of pathogens.

To prepare the photosensitizer composition, the one or more photoactive ingredients are typically exposed to a reducing agent, which is typically added to the photosensitizer composition or otherwise associated with the photoactive ingredient[s]. The reducing agent can include one or more reducing compounds. In general, efficient reducing compounds are atoms, molecules, compounds or the like in which the atoms have relatively large atomic radii, low electronegativity, and low ionization energy.

Desirable reducing compounds generally include, without limitation, ferrous ions, lithium aluminum hydride, hydrogen, sodium dithionite, zinc amalgam, potassium ferrocyanide, sodium borohydride, stannous ion, sulfites, hydrazine, diisobutylaluminum hydride, oxalic acid, ascorbic acid, ascorbate, active metal (e.g., potassium, calcium, sodium, barium or the like), reduced glutathione, dextrose, urea and any urea derivatives, anethol, glyoxol, ethylenediamine tetraacetic acid (EDTA), any combination thereof or the like. Preferably, the reducing agent is capable of reducing the photoactive ingredient[s] to a reduced form (e.g., a leuco form) either partially, substantially entirely (e.g., at least 90% reduced) or fully. It should be recognized, however, that it is undesirable to use excess reducing agent, which can thereafter prevent the photoactive ingredient from returning to a photoactive or oxidized state. A stoichiometric reduction can, in a preferred embodiment, be quantified visually or preferably spectrophotometrically as a percentage of color loss as the photoactive ingredient[s] transition from an oxidized form to a reduced form. It is contemplated that the photoactive ingredients can undergo a color loss of at least about 10%, more typically at least about 40% and possibly even at least about 70%, at least about 90% or even more. The amount of color loss can be determined by measuring optical density. In particular, the optical density of a photosensitizer solution or formulation can be determined non-quantitatively by visual inspection or quantitatively via a standard spectrophotometric assay. The formulation would be reduced to a condition in which the optical density is about 90% or less, more typically about 70% or less, still more typically about 50% or less an even possibly about 20% or about 10% or less of the original non-reduced form. If desirable, the solution can be reduced to the point where the optical density is essentially 0% (e.g., about 0.5% or less) of the original solution, a condition in which the formulation would appear virtually colorless.

The reducing agent can react with the photoactive ingredient[s] in a reaction (typically a reversible reaction) that causes electrons to be transferred from the reducing agent to the photoactive ingredient[s]. As one example, methylene blue and other dyes (e.g., phenothiazine dyes) can react with ascorbic acid such that electrons are taken on by the methylene blue or the like through a semiquinone intermediate, which then in turn dismutates to the reduced or leuco-form of the dye. The reducing agent is by contrast itself oxidized, for example ascorbic acid is converted to monodehydroascorbate and eventually dehydroascorbate. Neither the leuco-form of methylene blue, nor the oxidized forms of ascorbate are toxic *in vivo*, as both are commonly found intermediates in the normal metabolic breakdown of the parent compounds, are relatively stable and unreactive, and do not cause cellular damage. The reducing agent or the reducing agents contemplated can be relatively weaker or stronger in their ability to donate electrons to the photoactive ingredient[s] based upon their relative redox potential (measured in volts relative to a standard hydrogen electrode). Generally, reducing agents are more electronegative than the photoactive ingredients.

Once reduced, it is preferable for the photosensitizer composition and/or the photoactive ingredient[s] to lose at least some degree of color. Typically, when the photoactive ingredient is a dye such as those discussed herein and has an original dye color (e.g., blue) it will lose a substantial amount of color upon reduction of that ingredient. Such color loss can be due to, for example, saturation of the central thiazine ring in a phenothiazine compound, and resulting loss of pi-electron mobility. In a preferred embodiment, the one or more photoactive ingredient[s] lose at least about 50%, more typically at least about 80% and even more typically at least about 90% or more of their optical absorbance.

### Photodynamic Therapy

Photodynamic therapy can be performed according to the present disclosure by introducing the photosensitizer composition to tissue (e.g. human tissue, animal tissue, another substrate or otherwise) in a reduced and/or transportable state in which the at least one photoactive ingredient is typically less photo-active or substantially nonphotoactive. The photosensitizer composition, particularly the at least one photoactive ingredient thereof, is then allowed to pass through one or more barrier mediums to be adjacent, contacting or taken within ("collectively hereinafter referred to as "adjacent") a target medium, which preferably includes one or more pathogenic organisms (e.g., microbes, bacterial, virus, fungus, other nucleic-acid containing particle cells, or the like) or target cells (such as tumor cells). It should be understood that such barrier mediums can be within the target medium (e.g., cells). Once the at least one photoactive ingredient has penetrated through, or been associated with the barrier mediums, the at least one photoactive ingredient is then allowed to change form (e.g., become photoactive and/or oxidized) in the oxidizing microenvironment of the target medium. In addition or as an alternative to oxidization by the microenvironment of the target medium, or alternatively, the at least one photoactive ingredient may change form to its photoactive state optionally with the assistance of an oxidizing agent. The oxidizing agent can be any suitable compound that is more electropositive or oxidizing than the at least one photoactive ingredient in its reduced state. Examples of such an oxidizing agent include molecular oxygen, a hypochlorite solution, an oxidizing gas (e.g., fluorine, ozone, or the like), an oxidizing ion (e.g., permanganate ion or the like), an acid (e.g., nitric acid or the like), and a combination thereof. During and/or after such change in form, the photosensitizer composition, particularly the photoactive ingredients, are exposed to light such that the photoactive ingredients can then engage in photodynamic type I and/or II reactions and at least assist in inhibiting at least one pathogen associated with the target medium.

The photosensitizer composition can be applied to tissue using a variety of techniques. As examples, it can be sprayed on, brushed on, poured on or otherwise applied to the tissue. Moreover, the tissue to which the composition is applied can be human tissue, animal tissue or other living substrates or non-living substrates (e.g. surface of instruments) and can be performed for therapeutic purposes (e.g., for the purpose of inhibiting bacteria at a wound or lesion or elsewhere) for disinfection/sterilization procedures, experimental purposes or otherwise. In situations where the photosensitizer composition and/or the photoactive ingredients (e.g., photoactive dyes) thereof are substantially in a fully reduced state and/or are rendered less colorful or substantially colorless, light energy can pass through the solution without appreciable absorbance. Thus, in this state, the photosensitizer composition can be applied to a physical treatment site (e.g. a wound, lesion or otherwise) without significant local staining or discoloration. Furthermore, the optical density of the excess solution at the site is thereby reduced such that it does not substantially preclude the penetration of light into that treatment site.

It should be understood that the advantages of the solution or photoactive ingredients in terms of having less color can be achieved in situations where the photoactive ingredients were transported into and/or through a barrier medium and in situations where the ingredients did not experience such transport. Thus, the advantages of transportability and lesser color can be independent of each other or can depend on each other. As such, only one or the other or neither may be required for the invention, rather than both unless otherwise specified. It is also contemplated that neither advantage be required unless otherwise specified.

Generally, the photosensitizer composition and process of the present invention can be applied to a wide variety of target mediums. As examples, and without limitation, the target medium could be a wound (e.g., a surface wound), a lesion, a full thickness wound/incision, nasal mucosa, a nail bed, a cell or cells, bacteria, virus, fungus, or otherwise. For experimental purposes, the target can be almost any biological or synthetic substrate target mediums that will particularly benefit from the photosensitizer composition and process including, without limitation, periodontal pockets or other components of the oral mucosa.

Once applied, the photosensitizer composition and particularly the photoactive ingredients thereof may be transported to the target medium (e.g., a wound, lesion or other site containing bacterial cells to be inhibited) or may be applied directly to the target medium without additional transport being necessary. Generally, it is desirable for the photoactive ingredients in their transportable and/or lower color state (e.g., reduced state) to be applied to or transported to a location that is adjacent to cells of the target medium that are to be reduced and/or killed by a photodynamic reaction, as is described herein. As suggested, some of the photoactive ingredients may be directly applied to a location adjacent those cells and/or the photoactive ingredients can be transported (e.g., travel through, into or around) relative to one or more barrier mediums to the location adjacent those pathogens (e.g., cells or organisms). As used herein, a location adjacent the pathogens (e.g., cells) in this situation can mean that there is small space between the pathogens and the photoactive ingredients. It is preferable, however, that a location adjacent means that the photoactive ingredients are contacting or even more preferably taken up within the pathogens. In one preferred embodiment, the photoactive ingredients are internalized into cells (e.g., bacterial cells), either through transition into the aqueous cytosol partition, or through sequestration in the peptidoglycan bilayer membrane or LPS component.

The barrier mediums relative to which the photoactive ingredients are transported are typically environments that the photoactive ingredients might otherwise have difficulty penetrating if they were not in their transportable (lipophilic) or reduced state. In one embodiment, a barrier medium can be a hydrophilic or aqueous environment in which a normally polar photoactive ingredient might be very soluble if that ingredient were not in a reduced or otherwise transportable state. Such barrier mediums can be intracellular or extracellular. Examples of such barrier mediums include, without limitation, extracellular fluid, aqueous trans-membrane channels, aqueous vesicles, intracellular compartments, combinations thereof or the like. Advantageously, since the photoactive ingredients are in a transportable state, they can travel into, past or through the barrier mediums, which might otherwise impede their travel. Moreover, the photoactive ingredients may, in their transportable form, be more easily internalized into the cell and/or the cell's hydrophobic membrane components. This transportable form of the photoactive ingredient(s) may explain its increased antimicrobial efficacy against Gram-negative bacteria as demonstrated in Example III below. This increased antimicrobial efficacy against Gram-negative bacteria can be used to inhibit various Gram-negative pathogens including but not limited to *E. coli, Pseudomonas aeruginosa*, *Serratia marcescens*, black pigmented anaerobes such as *Porphyromonas gingivalis*, *Prevotella intermedia*, and *Prevotella nigrescens*, *Fusobacterium nucleatum*, *Aggregetobacter actinomycetemcomitans* or the like, and a combination thereof.

During or after location of the photoactive ingredients adjacent to the pathogens (e.g., cells) of the target medium, it is typically desirable for the photoactive ingredients to reach and/or return to a second, preferably photoactive state. Generally, it is contemplated that multiple different mechanisms may be employed to change the photoactive ingredients from their reduced state to a photoactive state. For example, chemicals such as oxidizers may be applied to the target medium for initiating the change. Alternatively energy may be provided to the photoactive ingredients in order to initiate the change. In one embodiment, photoactive ingredients that are in a reduced state are oxidized through a natural process of the target medium or cells thereof. For example, in cellular aqueous environments such as those discussed herein, the cell creates localized conditions to maintain a redox equilibrium within the cell. Such conditions can result in the oxidation of the reduced photoactive ingredients. In particular, oxidative conditions (created by intracellular atmospheric oxygen or other oxidizing agents) and/or metabolic pathways involving specific oxidative reactions act on the reduced photoactive ingredient to convert it back to its oxidized form. Where the photoactive ingredients are dyes (e.g., phenothiazine dyes or derivatives thereof such as tricyclic phenothiazines or methylene blue), the oxidation of those ingredients typically results in the ingredients becoming colorful or returning color from their colorless state. Preferably, the bulk of the photosensitizer composition that remains in the extracellular environment typically stays colorless, which can prevent staining, allow greater light penetration and can avoid undesirable photodynamic killing by photoactive ingredients that do not reach the target medium.

Once the photoactive ingredients become photoactive, light can be directed at the ingredients in order to achieve inhibition of the pathogen or cell-counts, against or within which the ingredients are located. In a preferred embodiment, the light energy activates the photoactive ingredients of a photosensitizer composition to either pass energy on directly to the cell or interact with molecular oxygen to produce reactive oxygen species for inhibiting the pathogen or cell (e.g., the bacterial cell). As used herein, light can include both visible and non-visible light. Moreover, such light can be provided by a variety of sources such as a light emitting diode (LED) a laser, incandescent source, OLED (organic light emitting diode), SLED (superluminescent light emitting diode), white light or filtered light or the like. One preferred source is a laser such as a non-thermal diode laser or HeNe laser emitting a wavelength matching one or more absorbance peaks of the partially or wholly oxidized state of the photosensitizer ingredient[s] being used. Such a laser typically has a total power output of 10 - 2000 milliwatts and preferably be in the range of 50-500 milliwatts.

It will be recognized that the process and photosensitizer composition of the present invention can provide for multiple advantages, although none of these advantages are required unless otherwise stated. As one advantage, selectivity of the photosensitized kill reaction between prokaryotes and eukaryotes can be increased. As another advantage, the lipophilicity ratio (log P) of the photoactive ingredient (e.g., the dye) can be adjusted such that enhanced uptake at prokaryotic biomembranes occurs, followed by internal conversion to a form more readily retained within the cell or organism. Yet another advantage of the invention is to permit selection of relatively arbitrary dye concentration, in order to maximize the number of peroxidative events at or within the prokaryotic biomembrane. Yet another advantage is to utilize a form of photosensitizer composition or photoactive ingredient that is well tolerated by humans. Other advantages inherent within the technique will become apparent to one skilled in the art of photodynamic inhibition of microbes or pathogens.

The following examples provided in accordance to the present invention are for illustrative purpose.

### Example I

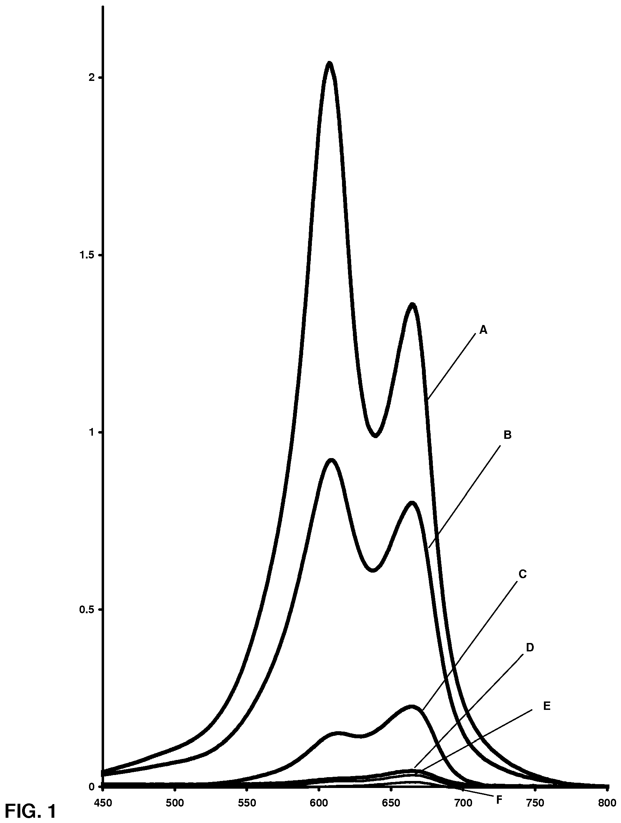

A 0.01% w/v methylene blue solution in an oxidized, cationic, or non-reduced state (hereinafter referred to as "non-reduced") was prepared by adding 1 ml of methylene blue solution USP (1%, ScholAR Chem) to 99 ml of sterile ultrapure water with mixing. Reduced methylene blue solutions for absorbance measurements were prepared using 0.05% w/v 0.1% w/v, 0.2% w/v, 0.5% w/v, and 1.0% w/v ascorbic acid. The reduced methylene blue solutions were transferred to sealed plastic tubes and the reduction/oxidation reaction was allowed to proceed overnight in the dark until completion. Absorbance measurements of these reduced methylene blue solutions were taken using thin film spectroscopy (1.0 mm path length) as shown in FIG. 1. FIG. 1 shows the absorbance (optical density) on its vertical axis and the range of wavelengths on its horizontal axis. FIG. 1 also shows the absorbance measurements for the non-reduced methylene blue solution (Line A in FIG. 1) and the reduced methylene blue solutions using 0.05% w/v (Line B in FIG. 1), 0.1% w/v (Line C in FIG. 1), 0.2% w/v (Line D in FIG. 1), 0.5% w/v (Line E in FIG. 1), and 1.0% w/v (Line F in FIG. 1) ascorbic acid. As shown in FIG. 1, the extent of reduction of methylene blue in each of the solutions was proportional to the amount of ascorbic acid added, and the data confirmed that the ending concentration of ascorbic acid in the solution was directly correlated to the reduction in optical density of methylene blue.

### Example II

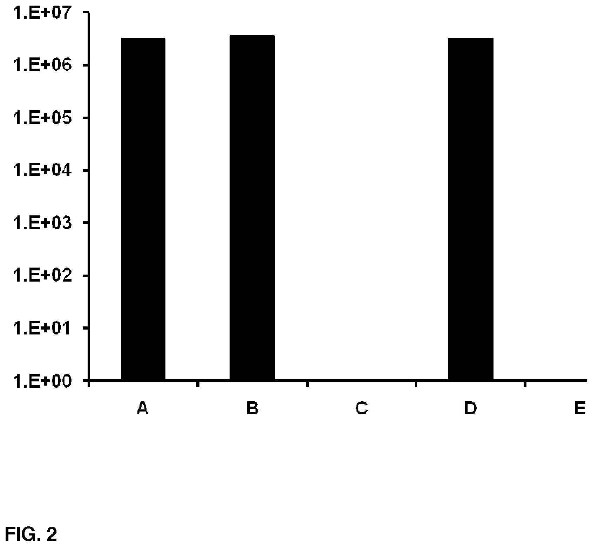

A 0.01% (v/v) aqueous solution of methylene blue was reduced substantially entirely to the reduced state using ascorbic acid. It was found that 0.2% (w/v) ascorbic acid was sufficient to render the solution virtually colorless in the visible range, and this was also confirmed by measuring the absorbance profile spectrophotometrically. This virtually colorless form of methylene blue solution was stable for several days when stored in a sealed container in the dark. The formulation was then tested for antibacterial efficacy against *E. Coli* (ATCC® 25922™) using two established photodynamic disinfection kill models (planktonic culture and biofilm) with a 670 nm laser energy source to provide desired illumination for 60 seconds. Referring to FIG. 2, results showed that there were substantial reductions in bacterial counts using the reduced formulation. FIG. 2 is a bar graph showing the eradication of *E. coli* expressed in log¹⁰ reduction in bacterial counts for the various samples used in the experiment. The "A" bar in FIG. 2 denotes the viable count of a bacterial suspension sample when treated with a phosphate buffered saline ("PBS") solution without illumination; the "B" bar represents the viable count of the bacterial suspension when treated with a solution of PBS and 0.2% w/v absorbic acid with illumination for 60 seconds; the "C" bar represents the viable count of the bacterial suspension when treated with a solution of 0.01% v/v methylene blue with illumination for 60 seconds; the "D" bar represents the viable count of the bacterial suspension when treated with a solution of 0.01% v/v methylene blue and 0.2% w/v absorbic acid without illumination; and the "E" bar represents the viable count of the bacterial suspension when treatment with the same solution as the "D" bar (i.e., the solution of 0.01% v/v methylene blue and 0.2% w/v absorbic acid) with illumination for 60 seconds. As shown in FIG. 2, the A, B and D bars showed limited, if any, antimicrobial efficacy as there was no detectable change in the viable count of the bacterial suspension before and after the application of these controls. The experimental controls showed that 0.2% w/v ascorbic acid did not itself demonstrate any antibacterial effects (either in PBS as shown in the B bar or in methylene blue as shown in the D bar). Furthermore, the spectrophotometric data for the reduced solution of methylene blue showed that there was little or no capacity for this solution to capture photons from a 670nm laser energy source. Thus, it would be expected that there would be little or no photodynamic inhibition seen when illuminating bacterial cells in the presence of substantially reduced methylene blue, since the photons would transmit through the solution rather than be absorbed and would therefore be unable to initiate photodynamic reactions such as the formation of excited energy states of the dye. However, in contrast to expected theoretical results, our results show that there is significant inhibition of *E. coli* when illuminated with a 670nm light source in the presence of reduced, colorless, photosensitizer as shown in the E bar in FIG. 2. Without being bound by theory, it is believed that a local interaction in the bacterial microenvironment causes the re-oxidation of reduced methylene blue back to the colored, cationic, light-absorbing blue form, thus allowing light energy to be captured and Type I and II photoreactions to be produced which are in turn inhibitory to the cell.

### Example III

In another in vitro experiment, inocula of each of the following microbes: methicillin-resistant *Staphylococcus aureus* or "MRSA" (ATCC® 33592™), methicillin-sensitive *Staphylococcus aureus* (ATCC® 25923™), *Staphylococcus epidermidis* (ATCC® 49461™), *Streptococcus mutans* (ATCC® 35668™), *E. Coli* (ATCC® 25922™), *Pseudomonas aeruginosa* (ATCC® 9027™), and *Serratia marcescens* (ATCC® 43862™), were prepared using PBS as the diluent and adjusted to ∼10⁷ CFU/ml via spectrophotometric measurement for 96-well planktonic assay. Also for the 96-well planktonic assay, mixed cultures of *Pseudomonas aeruginosa* and *Staphylococcus epidermidis* were also prepared by having each inoculum individually adjusted to ∼10⁷ CFU/ml and then combining 10 ml from each inoculum to produce a mixed 20 ml culture.

Two reduced 0.01% w/v methylene blue solutions were prepared. The first reduced methylene blue solution was reduced substantially (e.g., to a virtually colorless state) and was prepared by adding 40 mg ascorbic acid to 20 ml of 0.01% w/v oxidized methylene blue solution while stirring, for a final concentration of 0.2% w/v ascorbic acid ("Substantially Reduced Solution"). The second reduced methylene blue solution was partially reduced (e.g., to a slightly colored state) and was prepared by adding 10 mg ascorbic acid to 20 ml of 0.01% w/v methylene blue while stirring, for a final concentration of 0.05% w/v ascorbic acid ("Partially Reduced Solution"). The two solutions were transferred to sealed plastic tubes and the reduction/oxidation reaction was allowed to proceed overnight in the dark until completion.

Thereafter, the 96-well planktonic assay was prepared by combining a 100 µl aliquot of each of the inocula described above with 100 µl of test solution for a 1:1 ratio in test/control samples. The four test solutions used were the Substantially Reduced Solution, the Partially Reduced Solution, a non-reduced 0.01% w/v methylene blue solution ("Non-Reduced Solution"), and a 0.0005% w/v non-reduced (cationic) methylene blue solution ("Diluted Non-Reduced Solution"). The Diluted Non-Reduced Solution was formulated to be approximately optically equivalent to the Partially Reduced Solution. The Diluted Non-Reduced Solution was used in order to determine whether the antimicrobial activity of the Partially Reduced Solution was due to excitation of the non-reduced methylene blue fraction. Once the samples were prepared, the 96-well planktonic assay was then illuminated using a non-thermal 670 nm diode laser at 344 mW/cm² for 60 seconds (total energy dose = 20.6 Joules/cm²).

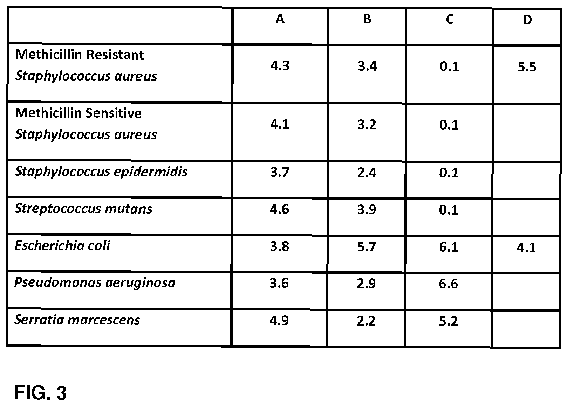

FIG. 3 is a table showing the data obtained from this experiment. The numbers provided in each column of FIG. 3 are the log₁₀ reductions in bacterial counts. Referring to FIG. 3, data showed that the illuminated Partially Reduced Solution (shown as column "B" in FIG. 3) and the Substantially Reduced Solution (shown as column "C" in FIG. 3) each exhibited a higher reduction of *E. coli* compared to the Non-Reduced Solution (shown as column "A" in FIG. 3) and the 0.0005% w/v methylene blue solution (shown as column "D" in FIG. 3). In contrast and as shown in FIG. 3, these two non-reduced methylene blue solutions (i.e., Non-Reduced Solution and Diluted Non-Reduced Solution) exhibited a higher reduction of MRSA compared to the reduced methylene blue solutions. It should be noted that the two non-reduced methylene blue solutions (i.e., Non-Reduced Solution and Diluted Non-Reduced Solution) showed similar antimicrobial activity (e.g., microbial reduction levels) against both MRSA and *E. coli.* These results suggested that the increased antimicrobial efficacy of the Partially Reduced Solution was not solely due to the fraction of non-reduced methylene blue molecule present.

Referring to FIG. 3, data also showed that the Substantially Reduced Solution provided selective antimicrobial efficacy against Gram-negative organisms (e.g., *E*. *coli*, *P*. *aeruginosa* and *S*. *marcescens*) when compared to Gram-positive organisms (e.g., MRSA, methicillin-sensitive *Staphylococcus aureus*, *S. epidermidis*, and *S*. *mutans*). In fact, the Substantially Reduced Solution provided virtually no antimicrobial efficacy against any of the Gram-positive samples. In contrast, the Non-Reduced Solution provided similar level of antimicrobial efficacy against both Gram-negative organisms and Gram-positive organisms. Furthermore, the Substantially Reduced Solution provided higher antimicrobial efficacy against Gram-negative organisms when compared to the Non-Reduced Solution. For example, as to *E*. *coli* and *P*. *aeruginosa*, the Substantially Reduced Solution samples demonstrated microbial reductions from control that were more than 2 log₁₀ (e.g., a factor of 100 times) greater than the Non-Reduced Solution samples.

Finally, data from the samples containing a mixture of *P*. *aeruginosa* and *S*. *epidermidis* provided further evidence of the selectivity of the Substantially Reduced Solution in eradicating Gram-negative bacteria expressed in log₁₀ reduction in bacterial counts. The data showed that the *P*. *aeruginosa* culture was completely eradicated (e.g., >6.8 log₁₀ reduction) by the Substantially Reduced Solution, yet the *S*. *epidermidis* population was left unaffected. In contrast, the Non-Reduced Solution provided similar level of antimicrobial efficacy against both *P. aeruginosa* (4.8 log₁₀ reduction) and *S*. *epidermidis* (3.8 log₁₀ reduction). The Non-Reduced Solution had a lower antimicrobial efficacy against *P. aeruginosa* (4.8 log₁₀ reduction) when compared to the efficacy level provided by the Substantially Reduced Solution against *P. aeruginosa* (>6.8 log₁₀ reduction).

### Example IV

An in vitro experiment was undertaken to assess the antibacterial efficacy of the reduced methylene blue described in Example I in a bacterial zone of inhibition photodynamic therapy model. Aqueous 0.01% w/v methylene blue was reduced substantially (substantially colorless) when combined with 0.2% w/v ascorbic acid as described in Example I. As previous experiments had demonstrated the selectivity of the reduced methylene blue against Gram-negative organisms as opposed to Gram-positive, the Gram-negative pathogen *Pseudomonas aeruginosa* was used as the test organism for this experiment. *P. aeruginosa* is a rod-shaped bacterium that plays a role in infectious conditions including otitis externa, infectious keratitis, pneumonia, and cystic fibrosis-related respiratory disease. It is also a significant factor in opportunistic infections of compromised sites such as wounds, burns, and surgical incisions.

During the in vitro experiment, a liquid inoculum of *P. aeruginosa* (∼10⁷ CFU/ml, ATCC#9027) was streaked over the entire surface of tryptic soy agar plates. These plates were allowed to dry. Thereafter, a non-reduced 0.01% w/v methylene blue solution was applied in a 20 µl aliquot directly onto the agar surface in a defined treatment site on the first quadrant of each of the plates; and the reduced 0.01% w/v methylene blue solution described in Example I was applied in a 20 µl aliquot directly onto the agar surface in a defined treatment site on the second quadrant of each of the plates. Defined treatment sites within third and fourth quadrants of each of the plates were used for dark controls (i.e., no illumination) to ensure that any reductions in microbial and/or bacterial viability were not due to the presence of methylene blue on the media surface during culture. Illumination was applied to each of the defined treatment sites of the first and second quadrants of the plates using a non-thermal 670 nm diode laser at a power density of 308 mW/cm² for 60 seconds (total energy dose = 18.5 Joules/cm²). Thereafter, all of the plates were placed back in culture at 37°C for 24 hours to allow viable bacteria to grow to visible confluence.

It was expected that in the event of bactericidal activity within each defined treatment site, a zone of no observable bacterial growth would be present at such defined treatment site after culture. Bacteria inoculated outside the defined treatment site should grow as normal and form a confluent lawn around the defined treatment site. If there was no observable difference between the defined treatment site and the non-treated area, it could then be concluded that the treatment administered did not lead to a reduction in bacterial viability. Zones of growth inhibition after treatment were scored visually and measured (diameter) using a calibrated electronic caliper.

The results of this experiment showed that the defined treatment sites treated with the reduced 0.01% w/v methylene blue solution and illumination, led to complete eradication of *Pseudomonas aeruginosa* within each of the treatment sites (9.7 mm treatment site with zero growth within the treatment site). In contrast, the treatment sites treated with the non-reduced 0.01% w/v methylene blue solution and illumination only caused a partial reduction in bacterial viability with many observable colonies of *P*. *aeruginosa* colonies growing within each of the treatment sites. No reduction in bacterial viability was observed in the dark controls with either the reduced or non-reduced methylene blue solution. These results further demonstrate the antimicrobial efficacy of reduced methylene blue against Gram-negative pathogens upon activation with visible light. Furthermore, the data suggests that reduced methylene blue provides greater antimicrobial efficacy than the non-reduced, cationic form of methylene blue.

Unless stated otherwise, particular chemistry or biology terms used herein are not intended to be restrictive of the invention. Plural components of the composition and/or steps of the process can be provided by a single components or step. Alternatively, a single component or step may be divided into separate plural components or steps.

## Claims

1. Use of a photosensitizer composition in the manufacture of a medicament for inhibiting at least one Gram-negative bacterial cell during photodynamic therapy comprising:
providing a photosensitizer composition having a phenothiazine in a reduced state wherein the phenothiazine in the reduced state has lost a degree of color and at least 10% of optical density compared to the phenothiazine in a photoactive state;
applying the photosensitizer composition to a substrate containing the at least one Gram-negative bacterial cell such that the phenothiazine in the a reduced state associates with at least one of the following: bacterial membrane structure of the at least one Gram-negative bacterial cell, intracellular environment of the at least one Gram-negative bacterial cell, and a combination thereof, and becomes oxidized to the photoactive state; and
exposing the phenothiazine to light at a wavelength absorbed by the phenothiazine so that the phenothiazine at least assists in inhibiting the at least one Gram-negative bacterial cell.

2. The use according to claim 1 wherein the at least one Gram-negative bacterial cell is selected from a group consisting of *E. coli, Pseudomonas aeruginosa, Serratia marcescens* and a combination thereof.

3. The use according to claim 1 wherein the at least one Gram-negative bacterial cell is selected from a group consisting of *Porphyromonas gingivalis, Prevotella intermedia, Prevotella nigrescens, Fusobacterium nucleatum, Aggregetobacter actinomycetemcomitans*, and a combination thereof.

4. The use according to any one of claim 1 to 3 wherein the phenothiazine is selected from a group consisting of methylene blue, toluidine blue O, a phenothiazine derivative, and a combination thereof.

5. The use according to any one of claim 1 to 4 wherein the step of providing the photosensitizer composition having the phenothiazine in a reduced state includes reducing the phenothiazine by a reducing agent causing the phenothiazine to lose the degree of color resulting in the photosensitizer composition losing at least 10% of the photosensitizer's composition's optical density.

6. The use according to claim 5 wherein the reducing agent is selected from the group consisting of ferrous ions, lithium aluminum hydride, hydrogen, sodium dithionite, zinc amalgam, potassium ferrocyanide, sodium borohydride, stannous ion, sulfites, hydrazine, diisobutylaluminum hydride, oxalic acid, ascorbic acid, ascorbate, an active metal, reduced glutathione, dextrose, urea, urea derivative, anethol, glyoxol, ethylenediamine tetraacetic acid (EDTA), and a combination thereof.

7. The use according to claim 5 or claim 6 wherein the photosensitizer composition's loss of optical density is at least 90%.

8. The use according to any one of claim 5 to 7 wherein the reducing agent includes ascorbic acid.

9. The use according to any one of claim 5 to 8 wherein the phenothiazine is 0.01% w/v methylene blue and the reducing agent is 0.2% w/v ascorbic acid.

10. The use according to any one of the preceding claims wherein the oxidation of the phenothiazine is assisted by application of an oxidizing agent.

11. The use according to claim 10 wherein the oxidizing agent is selected from the group consisting of molecular oxygen, a hypochlorite solution, an oxidizing gas, an oxidizing ion, an acid, and a combination thereof.

12. The use according to any one of the preceding claims wherein the light is provided by a light source selected from a group consisting of a laser, LED, incandescent source, OLED (organic light emitting diode), SLED (superluminescent light emitting diode), white light, filtered light, and a combination thereof.

## Patentansprüche

1. Verwendung einer Lichtsensibilisator-Zusammensetzung bei der Herstellung eines Medikaments zum Hemmen mindestens einer gramnegativen Bakterienzelle während einer photodynamischen Therapie, die umfasst:
Bereitstellen einer Lichtsensibilisator-Zusammensetzung, die über ein Phenothiazin in einem reduzierten Zustand verfügt, wobei das Phenothiazin in dem reduzierten Zustand einen Grad von Farbe und mindestens 10 % an optischer Dichte im Vergleich zu dem Phenothiazin in einem photoaktiven Zustand verloren hat;
Aufbringen der Lichtsensibilisator-Zusammensetzung auf ein Substrat, das die mindestens eine gramnegative Bakterienzelle enthält, so dass sich das Phenothiazin in dem reduzierten Zustand mit den folgenden Strukturen verbindet: einer Bakterienmembranstruktur der mindestens einen gramnegativen Bakterienzelle, der intrazellulären Umgebung der mindestens einen gramnegativen Bakterienzelle und/oder einer Kombination davon, und zu dem photoaktiven Zustand oxidiert wird; und
Belichten des Phenothiazins mit Licht einer Wellenlänge, die durch das Phenothiazin absorbiert wird, so dass das Phenothiazin die Hemmung der mindestens einen gramnegativen Bakterienzelle unterstützt.

2. Verwendung gemäß Anspruch 1, wobei die mindestens eine gramnegative Bakterienzelle aus der folgenden Gruppe ausgewählt wird: *E. Coli, Pseudomonas aeruginosa, Serratia marcescens* und einer Kombination davon.

3. Verwendung gemäß Anspruch 1, wobei die mindestens eine gramnegative Bakterienzelle aus der folgenden Gruppe ausgewählt wird: *Porphyromonas gingivalis, Prevotella intermedia, Prevotella nigrescens, Fusobacterium nucleatum, Aggregetobacter actinomycetemcomitans* und einer Kombination davon.

4. Verwendung gemäß einem der Ansprüche 1 bis 3, wobei das Phenothiazin aus der folgenden Gruppe ausgewählt wird: Methylenblau, Toluidinblau O, einem Phenothiazinderivat und einer Kombination davon.

5. Verwendung gemäß einem der Ansprüche 1 bis 4, wobei der Schritt eines Bereitstellens der Lichtsensibilisator-Zusammensetzung, in der das Phenothiazin in einem reduzierten Zustand vorliegt, umfasst: Reduzieren des Phenothiazins durch ein Reduktionsmittel, wodurch bewirkt wird, dass das Phenothiazin einen Grad von Farbe verliert, was dazu führt, dass die Lichtsensibilisator-Zusammensetzung mindestens 10 % ihrer optischen Dichte verliert.

6. Verwendung gemäß Anspruch 5, wobei das Reduktionsmittel aus der folgenden Gruppe ausgewählt wird: Eisenionen, Lithiumaluminiumhydrid, Wasserstoff, Natriumdithionit, Zinkamalgam, Kaliumferrocyanid, Natriumborhydrid, Zinnion, Sulfite, Hydrazin, Diisobutylaluminiumhydrid, Oxalsäure, Ascorbinsäure, Ascorbat, ein aktives Metall, reduziertes Glutathion, Dextrose, Harnstoff, Harnstoffderivat, Anethol, Glyoxol, Ethylendiamintetraessigsäure (EDTA) und eine Kombination davon.

7. Verwendung gemäß Anspruch 5 oder Anspruch 6, wobei der Verlust an optischer Dichte der Lichtsensibilisator-Zusammensetzung mindestens 90 % beträgt.

8. Verwendung gemäß einem der Ansprüche 5 bis 7, wobei das Reduktionsmittel Ascorbinsäure umfasst.

9. Verwendung gemäß einem der Ansprüche 5 bis 8, wobei das Phenothiazin 0,01 (w/v)%-iges Methylenblau und das Reduktionsmittel 0,2(w/v)%-ige Ascorbinsäure ist.

10. Verwendung gemäß einem der vorangehenden Ansprüche, wobei die Oxidation des Phenothiazins durch Anwendung eines Oxidationsmittels unterstützt wird.

11. Verwendung gemäß Anspruch 10, wobei das Oxidationsmittel aus der folgenden Gruppe ausgewählt wird: molekularer Sauerstoff, einer Hypochloritlösung, einem oxidierenden Gas, einem oxidierenden Ion, einer Säure und einer Kombination davon.

12. Verwendung gemäß einem der vorangehenden Ansprüche, wobei das Licht durch eine Lichtquelle zur Verfügung gestellt wird, die aus der folgenden Gruppe ausgewählt wird: einem Laser, LED, Glühlampe, OLED (organische Leuchtdiode), SLED (Superlumineszenzdiode), Weißlicht, gefiltertes Licht und eine Kombination davon.

## Revendications

1. Utilisation d'une composition photosensibilisante dans la fabrication d'un médicament destiné à inhiber au moins une cellule bactérienne à Gram négatif pendant une thérapie photodynamique, comprenant :
fournir une composition photosensibilisante ayant une phénothiazine dans un état réduit dans laquelle la phénothiazine à l'état réduit a perdu un degré de couleur et au moins 10 % de densité optique par rapport à la phénothiazine dans un état photoactif,
appliquer la composition photosensibilisante à un substrat contenant la au moins une cellule bactérienne à Gram négatif de telle sorte que la phénothiazine à l'état réduit s'associe à au moins un des éléments suivants : structure de membrane bactérienne de la au moins une cellule bactérienne à Gram négatif, environnement intracellulaire de la au moins une cellule bactérienne à Gram négatif et une combinaison de ceux-ci, et s'oxyde à l'état photoactif, et
exposer la phénothiazine à une lumière à une longueur d'onde absorbée par la phénothiazine de telle sorte que la phénothiazine aide au moins à inhiber la au moins une cellule bactérienne à Gram négatif.

2. Utilisation selon la revendication 1, dans laquelle la au moins une cellule bactérienne à Gram négatif est choisie parmi un groupe constitué de *E. coli, Pseudomonas aeruginosa, Serratia marcescens* et d'une combinaison de celles-ci.

3. Utilisation selon la revendication 1, dans laquelle la au moins une cellule bactérienne à Gram négatif est choisie parmi un groupe constitué de *Porphyromonas gingivalis, Prevotella Intermedia, Prevotella nigrescens, Fusobacterium nucleatum, Aggregetobacter actinomycetemcomitans* et d'une combinaison de celles-ci.

4. Utilisation selon l'une quelconque des revendications 1 à 3, dans laquelle la phénothiazine est choisie parmi un groupe constitué de bleu de méthylène, de bleu de toluidine O, d'un dérivé de phénothiazine et d'une combinaison de ceux-ci.

5. Utilisation selon l'une quelconque des revendications 1 à 4, dans laquelle l'étape consistant à fournir la composition photosensibilisante ayant la phénothiazine dans un état réduit inclut la réduction de la phénothiazine par un agent réducteur amenant la phénothiazine à perdre le degré de couleur, le résultat étant que la composition photosensibilisante perd au moins 10 % de la densité optique de la composition photosensibilisante.

6. Utilisation selon la revendication 5, dans laquelle l'agent réducteur est choisi parmi le groupe constitué d'ions ferreux, d'hydrure de lithium et d'aluminium, d'hydrogène, de dithionite de sodium, d'amalgamme de zinc, de ferrocyanure de potassium, de borohydrure de sodium, d'ion stanneux, de sulfites, d'hydrazine, d'hydrure de diisobutylaluminium, d'acide oxalique, d'acide ascorbique, d'ascorbate, d'un métal actif, de glutathion réduit, de dextrose, d'urée, de dérivé d'urée, d'anéthol, de glyoxol, d'acide éthylène diamine tétra-acétique (EDTA) et d'une combinaison de ceux-ci.

7. Utilisation selon la revendication 5 ou la revendication 6, dans laquelle la perte de densité optique de la composition photosensibilisante est au moins de 90 %.

8. Utilisation selon l'une quelconque des revendications 5 à 7, dans lequel l'agent réducteur inclut de l'acide ascorbique.

9. Utilisation selon l'une quelconque des revendications 5 à 8, dans laquelle la phénothiazine est le bleu de méthylène à 0,01 % en p/v et l'agent réducteur est l'acide ascorbique à 0,2 % en p/v.

10. Utilisation selon l'une quelconque des revendications précédentes, dans laquelle l'oxydation de la phénothiazine est favorisée par l'application d'un agent oxydant.

11. Utilisation selon la revendication 10, dans laquelle l'agent oxydant est choisi parmi le groupe constitué d'oxygène moléculaire, d'une solution d'hypochlorite, d'un gaz oxydant, d'un ion oxydant, d'un acide et d'une combinaison de ceux-ci.

12. Utilisation selon l'une quelconque des revendications précédentes, dans laquelle la lumière est fournie par une source de lumière choisie parmi un groupe constitué d'un laser, d'une LED, d'une source à incandescence, d'une OLED (diode électroluminescente organique), d'une SLED (diode super-luminescente), d'une lumière blanche, d'une lumière filtrée et d'une combinaison de ceux-ci.
